# EUROPEAN PATENT SPECIFICATION

(11) **EP 2 347 016 B1**
(45) Date of publication and mention of the grant of the patent: **03.12.2014**
(21) Application number: 08874967.6
(22) Date of filing: 10.10.2008
(51) Int. Cl.: C12Q 1/68

(54) **METHOD FOR DETERMINING THE RESISTANCE STATUS OF FUNGI AND YEASTS, IN PARTICULAR OF ASPERGILLUS FUMIGATUS**
VERFAHREN ZUR BESTIMMUNG DES RESISTENZSTATUS VON PILZEN UND HEFEN, INSBESONDERE ASPERGILLUS FUMIGATUS
PROCÉDÉ POUR LA DÉTERMINATION DE L'ÉTAT DE RÉSISTANCE DE CHAMPIGNONS ET DE LEVURES, EN PARTICULIER D'ASPERGILLUS FUMIGATUS

(43) Date of publication of application: 27.07.2011
(73) Proprietor: Stichting Ter Bevordering Van De Farmacodynamiek, 6602 HV Wijchen (NL)
(72) Inventor: KLAASSEN, Cornelis, Hendrikus, Wilhelm, NL-6605 DD Wijchen (NL)
(74) Representative: Laenen, Bart Roger Albert
(86) International application number: PCT/EP2008/008577
(87) International publication number: WO 2010/040374

(56) References cited:
- EP-A2- 1 362 928
- WO-A-98/17825
- US-A1- 2006 127 934
- GARCIA-EFFRON G ET AL.: "Rapid detection of triazole antifungal resistance in Aspergillus fumigatus" JOURNAL OF CLINICAL MICROBIOLOGY, vol. 46, no. 4, April 2008 (2008-04), pages 1200-1206, XP002545937 cited in the application
- BALASHOV S V ET AL.: "Rapid, high-throughput, multiplex, real-time PCR for identification of mutations in the cyp51A gene of Aspergillus fumigatus that confer resistance to itraconazole" JOURNAL OF CLINICAL MICROBIOLOGY, vol. 43, 2005, pages 214-222, XP002545938
- RODRIGUEZ-TUDELA J L ET AL.: "Epidemiological cutoffs and cross-resistance to azole drugs in Aspergillus fumigatus" ANTIMICROBIAL AGENTS AND CHEMOTHERAPY, vol. 52, no. 7, July 2008 (2008-07), pages 2468-2472, XP002545939 -& DATABASE EMBL [Online] EUROPEAN MOLECULAR BIOLOGY LABORATORY; 6 July 2008 (2008-07-06), RODRIGUEZ-TUDELA J L ET AL.: "Epidemiological cutoffs and cross-resistance to azole drugs in Aspergillus fumigatus" XP002545943 Database accession no. EU626235
- MELLADO ET AL.: "A new Aspergillus fumigatus resistance mechanism conferring in vitro cross-resistance to azole antifungals involves a combination of cyp51A alterations" ANTIMICROBIAL AGENTS AND CHEMOTHERAPY, vol. 51, no. 6, June 2007 (2007-06), pages 1897-1904, XP002545940
- VERWEIJ P E ET AL.: "Multiple-triazole-resistant aspergillosis" NEW ENGLAND JOURNAL OF MEDICINE, vol. 356, no. 14, 5 April 2007 (2007-04-05), pages 1481-1483, XP002545941
- HUNG ET AL: "Genotyping of the G1138A mutation of the FGFR3 gene in patients with achondroplasia using high-resolution melting analysis" CLINICAL BIOCHEMISTRY, ELSEVIER INC, US, CA, vol. 41, no. 3, 31 January 2008 (2008-01-31), pages 162-166, XP022442534 ISSN: 0009-9120
- SNELDERS E ET AL.: "Emergence of azole resistance in Aspergillus fumigatus and spread of a single resistance mechanism" PLOS MEDICINE, vol. 5, no. 11, November 2008 (2008-11), pages 1629-1637, XP002545942
- M. Erali: "Multiplex Single-Color PCR with Amplicon Melting Analysis for Identification of Aspergillus Species", Clinical Chemistry, vol. 52, no. 7, 1 July 2006 (2006-07-01), pages 1443-1445, XP055052342, ISSN: 0009-9147, DOI: 10.1373/clinchem.2006.068510
- WILLMORE-PAYNE C ET AL: "Human malignant melanoma: detection of BRAF- and c-kit-activating mutations by high-resolution amplicon melting analysis", HUMAN PATHOLOGY, SAUNDERS, PHILADELPHIA, PA, US, vol. 36, no. 5, 1 May 2005 (2005-05-01), pages 486-493, XP004916982, ISSN: 0046-8177, DOI: 10.1016/J.HUMPATH.2005.03.015
- WITTWER C T ET AL: "High-resolution genotyping by amplicon melting analysis using LCGreen", CLINICAL CHEMISTRY, AMERICAN ASSOCIATION FOR CLINICAL CHEMISTRY, WASHINGTON, DC, vol. 49, no. 6 Pt 1, 1 June 2003 (2003-06-01), pages 853-860, XP002330143, ISSN: 0009-9147, DOI: 10.1373/49.6.853

## Description

### FIELD OF THE INVENTION

The invention relates to a method for determining the resistance status of fungi and yeasts, in particular of *Aspergillus fumigatus* as present in a patient suspected of having an invasive infection. The invention further relates to a method for detecting mutations in the genome of fungi and yeasts, in particular in *Aspergillus fumigatus.* The invention also provides probes, primers and kits for use in the method.

### BACKGROUND OF THE INVENTION

*Aspergillus fumigatus* is the most common fungus involved in invasive fungal infections and can lead to life-threatening complications in immunocompromized individuals. Treatment of an infection by this fungus often involves triazole based antifungal agents such as fluconazole, voriconazole, itraconazole, posaconazole and others. Resistance of A. *fumigatus* against one or more of these antifungal agents has been described and involves either a mutation at a glycine residue at position 54, a mutation at a glycine residue at position 138 or a mutation at a methionine residue at position 220, all in the A. *fumigatus* cyp51A gene. These positions have independently been implicated in the resistance phenotype.

Several other amino acid substitutions have been described at other positions in the cyp51A gene but their significance with respect to naturally occurring phenotypically resistant isolates is still questionable. These are mutations that have been found in the laboratory after exposing sensitive isolates to antifungal compounds or for instance following random mutagenesis. Such other positions involve for instance Gly448 and Asn22.

Recently A. *fumigatus* isolates have been reported that are resistant to multiple of these triazole based antifungal compounds. The molecular mechanism responsible for this resistance has been elucidated and involves two specific nucleotide sequence alterations, both in the *cyp*51A gene. The first is a duplication of a 34 bp region in the promoter region of this gene. The sequence of this 34 bp region is GAATCACGCGGTCCGGATGTGTGCTGAGCCGAAT. The second alteration is a mutation of codon 98 where a leucine is changed into a histidine. Each of the two mutations alone does not lead to the resistance phenotype, but together they lead to significantly increased MIC levels for multiple triazole based antifungal compounds.

Interestingly, the inventors have found that naturally occurring isolates carrying only one of these two mutations are not found in the A. *fumigatus* population, so determination of the presence of either of the two mutations should be indicative for the multi-resistance phenotype.

Recently it has become clear that the prevalence of multi-triazole resistant A. *fumigatus* is increasing. Moreover, it has been established that individuals can be colonized by multiple different A. *fumigatus* isolates at the same time. In patients with invasive aspergillosis only one of these isolates has become invasive. This means that even if multiple isolates are cultivated and tested for resistance, the test result does not necessarily represent the resistance status of an isolate that may have become invasive.

At present, diagnosis of invasive aspergillosis by a multi-triazole resistant A. *fumigatus* isolate requires cultivation of the fungus from clinical samples and phenotypic testing for resistance (M. Erali, Clinical Chemistry, vol.52, no. 7, 1 July 2006, P. 1443-1445; Garcia-Effron G. et al., J. Clin. Microbiol., vol. 46, No. 4, April 2008, P. 1200-1206; Balashov S.H. et al., J. Clin . Microbiol., vol. 43, 2005, P. 214-222). The presence of multiple genotypically or phenotypically different isolates in respiratory samples may complicate this diagnosis if the actual invading isolate has not been identified. To maximize the chances of finding a resistant isolate (if present) in respiratory samples such as sputa or broncho-alveolar lavages or other patient samples, multiple individually cultivated isolates need to be analyzed. This may impose a serious burden on laboratory personnel.

In addition, when the invading isolate is a multi-triazole resistant isolate but is not immediately recognized as such the patient may receive improper treatment. The ultimate consequence thereof may be that the patient dies.

The problems and principles that are described above for *Aspergillus fumigatus* also apply to other fungi and yeasts.

### SUMMARY OF THE INVENTION

It is therefore a first general object of the invention to provide a new method for determining the resistance status of fungi and yeasts. It is a further, more specific object of the invention to provide such method for determining the resistance status of *Aspergillus fumigatus* with respect to triazole based antifungal compounds. In this respect "determining the resistance status" is intended to mean "determining if a fungal isolate is sensitive or resistant to antifungal compounds of the class of triazole based antifungal compounds".

Typically, identification of mutations leading to resistance against triazoles in A. *fumigatus* is performed using DNA sequence analysis of the *cyp*51A gene. Since this technique requires multiple post-PCR steps (involving very large quantities of PCR products), these methods are also more prone to contamination and thus require rigorous protocols and dedicated pre- and post PCR facilities. The same applies to the analysis of resistance related mutations in other fungi and yeasts.

Obviously, any additional post-PCR step also increases the workload and is associated with higher personnel costs and increased turn-around time. The switch to real-time PCR formats involving the use of fluorescently labelled oligonucleotides may overcome most of these disadvantages. However, use of fluorescent probes is by no means a guarantee to success. Sometimes, for no obvious reasons, assays based on fluorescent probes fail to yield an adequate signal and may need to undergo extensive optimization before robust amplification and mutation detection is achieved. Further prior art methods require a preamplification step (EP 1 362 928); use DNA isolated from tissue samples, such as tumor samples (Willmore-Payne C. et al., Human Pathology, Saunders, Philadelphia, PA, US, vol. 36, no. 5, 1 May 2005, p 486-493); depart from genomic DNA extracted from dried blood spots (Wittmer C. et al., Clinical Chemistry, vol. 49, no. 6 Pt 1, 1 June 2003, p 853-860)

It is therefore a further object of the invention to provide a less complex detection assay format that requires only one or even no fluorescent oligonucleotides at all.

In the research that led to the invention it was found that a molecular analysis of circulating cell-free DNA in serum directly focuses the diagnosis on the actual invading isolate and thus leads to a rapid diagnosis of (multi-)triazole resistant invasive aspergillosis.

The isolate that is found in serum is the actual invading isolate and thus gives the most direct and relevant information that is needed for optimal patient treatment. Until the present invention, however, analysis has been done on isolates that were first isolated from other body fluids, such as for example sputum or BAL, and subsequently cultured. Such isolate is not necessarily the invading one. According to the invention, fungal or yeast DNA is now directly isolated from blood-fractions (such as serum or plasma) and tested for the presence of mutations.

The invention thus provides a method for determining the resistance status of *Aspergillus fumigatus* in a body sample of a patient suspected of having an invasive infection, comprising the steps of:
a) isolating DNA from a body sample of the patient,
b) determining the presence of a mutation(s) in a DNA region of *Aspergillus fumigatus* that potentially comprises a 34 bp promoter duplication, and
c) correlating the resistance status of the fungus or yeast to the mutations found,
wherein the body sample is blood or a blood derivative, and is in particular serum.

The DNA can be total DNA isolated from the body sample or can be specific yeast or fungus DNA that can be isolated from the total DNA by capture with a probe that is specific for the DNA of the yeast or fungus of which the resistance status is to be detected.

The resistance status correlates to the mutations found. When the DNA of the *Aspergillus fumigatus* isolate carries a duplication in the promoter of the *cyp*51A gene and a mutation that results in a change of the codon encoding the leucine residue at position 98 in the CYP51A protein into another amino acid, in particular histidine, the isolate is multi-triazole resistant. The duplication preferably results in a tandem repeat of the sequence GAATCACGCGGTCCGGATGTGTGCTGAGCCGAAT. It can however not be excluded that other duplications or changes in the promoter region have the same effect. Such other changes are also part of this invention.

When the DNA of the *Aspergillus fumigatus* isolate that is tested carries a mutation that results in replacement of the codon encoding the glycine residue at position 54 in the CYP51A protein by another amino acid, in particular glutamic acid or valine, the isolate is triazole-resistant.

In case the DNA of the *Aspergillus fumigatus* strain carries a mutation that results in replacement of the codon encoding the methionine residue at position 220 in the CYP51A protein by another amino acid, in particular valine or lysine, the isolate is triazole-resistant.

When the DNA of the *Aspergillus fumigatus* isolate carries a mutation that results in replacement of the codon encoding the glycine residue at position 138 in the CYP51A protein by another amino acid, in particular cysteine, the isolate is multi-triazole-resistant

In order to detect the resistance status of the *Aspergillus fumigatus isolate* that actually invaded the body and to be able to tailor the treatment to that particular strain it is most preferred to use serum as a sample. However, blood and products derived therefrom other than serum, in particular plasma can also be used. Suitably DNA is isolated from the sample, such as serum, and used in any method that can detect the presence of mutations. Such methods are for example DNA sequence analysis, restriction enzyme analysis, hybridisation based assays (such as in-line probe assays, enzyme immunoassays, DNA microarrays etc.). Preferably, the method is performed in a closed-tube format, such as by means of real-time PCR. This way contamination and multiple handling steps are avoided.

Real-time PCR has revolutionized the field of molecular diagnostics. The ability to perform amplification, detection and quantitation of specific DNA/RNA targets in a single closed-tube format has several obvious advantages over conventional PCR approaches in terms of speed, throughput, reduced susceptibility to contamination etc.

Real-time PCR approaches to detect specific nucleotide sequence variations usually employ fluorescent probes for the interrogation of a specific (part of a) target sequence. Several fluorescent formats have been described but probably the most widespread approaches use either hybridization probes (in particular FRET probes) or hydrolysis probes (a.k.a. in particular TaqMan probes), each with specific advantages and disadvantages over the other. Recently, molecular beacons have been described for detection of mutations leading to triazole resistance in A. *fumigatus* (Garcia-Effron G. et al., J Clin Microbiol. 46(4):1200-6 (2008). This assay is performed on respiratory fluids such as sputum or BAL. This method does not necessarily lead to the detection of the actual invading A. *fumigatus* isolate.

High-resolution melting (HRM) is a novel addition to the field of real-time PCR analysis of DNA nucleotide sequence variations such as point-mutations, single-nucleotide polymorphisms (SNP's) and insertions/deletions. Central in the application of HRM is a fluorescent dye of which the fluorescence is greatly enhanced in the presence of double stranded DNA. The melting behavior of the amplified product is analyzed by the generation of melting curves (measuring the fluorescence of this dye as a function of increasing temperature). Alternatively, melting peaks are analyzed by taking the (negative) first derivative of the melting curve data. Different melting curves are obtained as a result of nucleotide sequence variations and serve as a basis for identification of samples with nucleotide sequence alterations. HRM eliminates the need for specific fluorescent oligonucleotide probes and thus further reduces the costs associated with and complexity of real-time mutation detection assays.

According to the invention three new assay formats were developed based on the above described techniques. These techniques are suitably used for analysing cell-free DNA from invading fungi or yeasts, in particular from *Aspergillus fumigatus,* as found in body fluids, in particular serum.

According to a first aspect thereof, the invention provides a method for detecting mutations in fungi and yeasts, comprising the steps of:
a) providing a body sample;
b) amplifying a target nucleic acid that is present in the sample, using a primer set for amplification of a DNA region of *Aspergillus fumigatus* that potentially comprises a 34 bp promoter duplication, which primer set comprises the forward primer:
   5'-AATAATCGCAGCACCACTCC-3' **(SEQ ID NO:1)**
      and the reverse primer
   5'-TGGTATGCTGGAACTACACCTT-3' **(SEQ ID NO:2);**
c) allowing double stranded nucleic acid to form between amplified target nucleic acid and reference nucleic acid that is at least partially complementary to the target nucleic acid , in the presence of a fluorescent compound, the fluorescence of which is enhanced or altered upon intercalation into double stranded nucleic acid;
d) melting the double stranded nucleic acid thus formed and measuring the fluorescence in the sample as a function of the temperature to obtain a melting curve, and
e) analysing the melting curve to detect mutations in the target nucleic acid sequence.

The invention provides two variants of this general method.

In the first variant, which is called herein the "probe variant" or "single probe variant" the reference nucleic acid is a probe which is normally smaller than the target nucleic acid. The probe is labelled and the label is excited by the fluorescence that is emitted by the fluorescent compound that is intercalated into double stranded nucleic acid. Thus, upon melting the double strand the signal emitted by the label on the probe will decrease. This is visible in the melting curve. Depending on the probe and the target, there may be either one or more mismatches in the double stranded probe-target combination or not. The duplexes with a mismatch will melt at a lower temperature than the duplexes without mismatch.

In the second variant there is no labelled probe but a reference sequence that will normally be of about the same size as the target nucleic acid. The target nucleic acid and the reference nucleic acid are amplified in the same amplification reaction. After amplification is complete the double stranded amplicons are melted and reannealed. This will lead to homoduplexes between target and target and between reference and reference and to heteroduplexes between target and reference. In case the heteroduplex comprises a mismatch because either the target or the reference comprises a mutation, the heteroduplex will melt at a lower temperature than the homoduplexes. This variant is called herein the HRM variant.

According to a further aspect thereof, the presence of a mutation is deduced from the shape of the melting peak of the amplified product. This eliminates the need for either the HRM variant or the single probe variant. This embodiment of the method of the invention will be called herein the "amplicon variant".

### DETAILED DESCRIPTION OF THE INVENTION

The (HRM variant) of the detection method of the invention is based on the formation of DNA heteroduplexes that can be formed by a denaturation and re-annealing step following the simultaneous amplification of target and reference DNA in a single tube. In this variant, the nucleic acid that is at least partially complementary to the target nucleic acid is an amplified reference nucleic acid. When the fungus is *Aspergillus fumigatus,* the DNA sequence of the reference sequence is for example wild type *Aspergillus fumigatus* DNA.

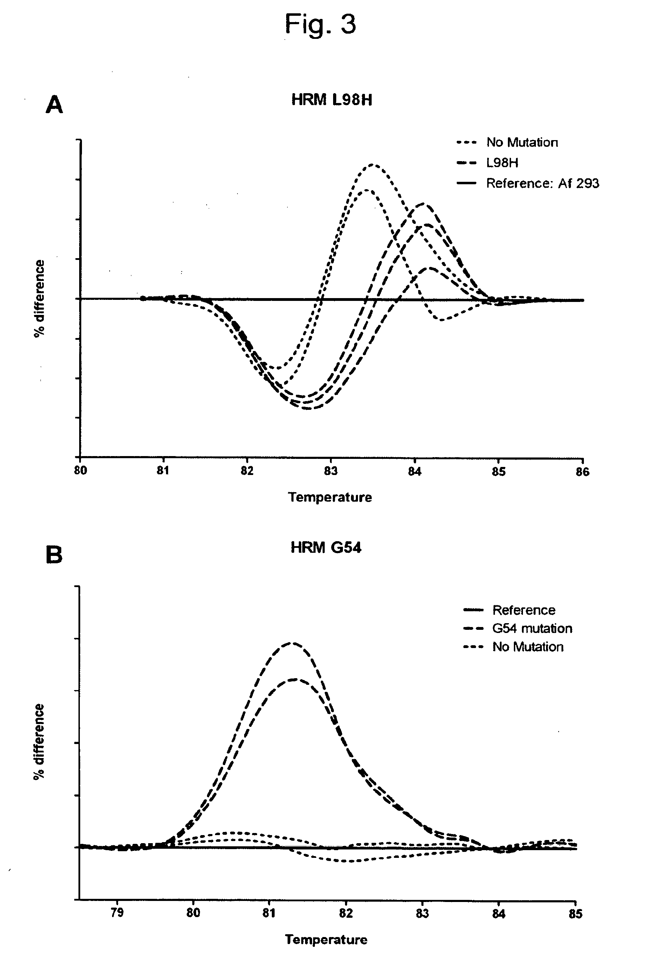

The presence of a mutation in the target sequence will lead to a local mismatch in the heteroduplex molecule that will result in a different melting behaviour of that mismatched heteroduplex and can be analyzed by the generation of melting curves. Analysis can be based on a melting curve itself or on the first derivative thereof or on a difference plot (see for example **Figure 3****).**

In order to create the heteroduplexes, prior to amplification, samples are spiked with a reference sample (e.g. a confirmed wild-type sample) to identify any nucleotide polymorphism. Alternatively, it can be spiked with a sample containing a known mutation to discriminate between samples samples having the same mutation as the reference and samples having no or another mutation.

Alternative to the simultaneous amplification of target and reference DNA, heteroduplexes can be created post-amplification by combining the amplified product and a reference amplified product or a reference product that is generated otherwise.

In the probe variant, the detection method is a targeted approach towards specific regions of the *cyp*51A gene. A fluorescently labeled probe is used to cover the part of the target region containing the mutation. Excitation of this probe is achieved indirectly through the presence of the fluorescent intercalating compound, such as the HRM dye, in the partially double stranded DNA that is formed upon hybridization of the probe to the target nucleic acid.

Based on the melting behavior of this probe when subjected to increasing temperatures, the presence or absence of a mutation underneath the probe is easily deduced from the resulting melting data. Presence of a mutation will for example become apparent when the negative first derivative of the melting curve has a peak at a lower temperature than wild-type products if the probe is complementary to the wild-type sequence. Alternatively, when the probe contains the mutation, the peak is visible at a higher temperature.

According to the amplicon variant, amplicons are generated and their melting curves analysed. This method does not require a probe or reference sequence being present in the same amplification reaction as the target. The melting curve of the target amplicon is compared with the melting curve of a reference amplicon prepared in a separate amplification reaction. The target amplicon will show different melting behaviour than the reference amplicon when a duplication or certain mutations are present in the target.

Alternatively, it is possible in this method to have the amplification of the reference sequence take place in the same tube as the target. However, formation of heteroduplexes between target and reference are to be avoided. Thus, the melting curves are prepared after the last amplification step has taken place and without a further melting steps such as the one used in the HRM variant. This variant is called herein the "amplicon variant".

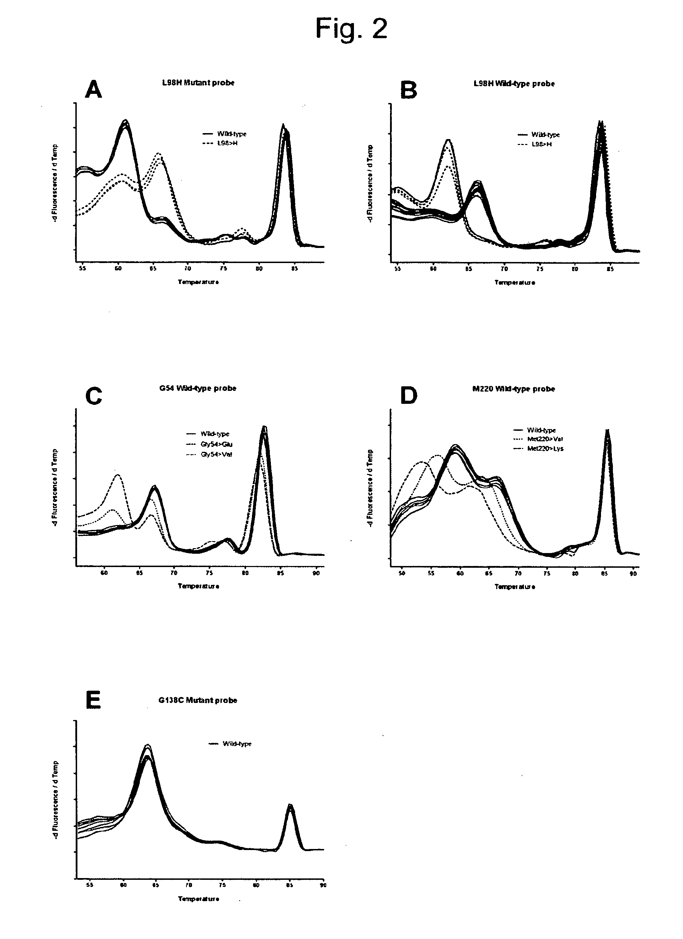

For instance, the presence of a mutation at the G54 position can also be deduced from the shape of the melting peak at 75-85°C **(****figure 2C****).**

Preferably the methods as described herein are used for detecting mutations in genes of the fungus *Aspergillus fumigatus.*

The body sample can be blood or a blood derivative or a sample of an organ, and is preferably serum. Preferably, DNA is isolated from the body sample before amplifying the target sequence comprised therein.

Serum contains DNA originating from the actual invading isolate. Multiple *Aspergillus fumigatus* isolates may be colonizing the patient, but the invading isolate is the one that must be treated. Analysis of DNA from the patient's serum thus guarantees that the correct treatment can be selected that is effective for the isolate involved.

Suitably, the amplification is performed by means of real-time PCR and in particular uses intercalating dyes.

The fluorescent compound, the fluorescence of which is enhanced or altered upon intercalation into double stranded nucleic acid, is suitably a dye selected from but not limited to the HRM dye (e.g. Resolight Dye or HRM Dye, Roche Diagnostics), Sybr-Green, LC-Green (Idaho technologies), LC-Green plus, SYTO-dyes (Invitrogen) and others.

In the amplicon variant, the amplification reaction can be performed using symmetrical conditions which could lead to an improved limit of detection.

In a regular real-time PCR assay using fluorescent probes, each probe has to compete with the complementary DNA strand in order to anneal to the target strand. In the probe variant, in order to maximize the amount of probes that can hybridize to the target and to minimize the amount of double-stranded PCR product, the PCR is performed in asymmetrical fashion, preferentially amplifying the strand to which the probe should anneal.

For optimal detection of DNA mutations that are indicative of the resistance status of the isolate to be analysed using the HRM variant, the target and reference sequence are preferably present in approximately equimolar amounts. This requires that the amount of target DNA that is used in an amplification reaction is first estimated or quantitated.

However, if DNA is isolated from a body fluid, it may be difficult to determine precisely how many target sequence is present. Therefore, in a preferred embodiment, the amount of target DNA that is isolated from a body fluid is limited by using a DNA isolation procedure that yields a predetermined amount of DNA. This way, the HRM variant can be performed without the need for quantification of the target DNA. This can for instance be established by using specified amounts of capture probes and/or by using DNA isolation procedures that have a specified capacity of DNA isolation.

The method as described here can also be performed with DNA from other sources than blood products, for example isolated from cultured isolates.

With these techniques, the formation of aberrant melting profiles is only indicative for the presence of a nucleotide polymorphism but not for its identity. There is always a chance that two different polymorphisms will lead to similar changes in melting peak. Therefore, according to a preferred embodiment, mutation specific probes are used in the probe variant or reference sequences with known mutations in the HRM variant to verify the identity of the polymorphism.

Alternatively, DNA sequence analysis of the samples with aberrant melting profiles can be performed.

The different variants of the invention as described above have specific advantages and disadvantages over each other. The probe variant is restricted to specific parts of the target that are covered by the probe. By using a wild-type probe, polymorphisms underneath the probe may be detected. The identity of any polymorphism then can be established using other approaches. If one is only interested in specific mutations, probes matching the specific mutation can be used.

The HRM variant may identify all nucleotide polymorphisms in the amplified product, but this includes the insignificant ones that do not result in an amino acid change.

In the HRM variant, by using partially overlapping amplicons, an entire gene can rapidly be screened for the presence of mutations *per se.*

In the probe variant, separate amplification reactions can be used to analyze the different codons of the *cyp*51A gene that are involved in a resistance phenotype. In another embodiment, multiple positions can be analyzed in a single reaction by choosing different fluorescent labels for each of the individual mutations, or by modifying the length or composition of the probes such that the distinction can be made based on the location of the melting peaks. In yet another embodiment, longer DNA fragments are amplified that may contain more target positions. In another embodiment, combinations of the above are used.

Interestingly and unexpectedly, the probe variant comes with a built-in internal amplification control. A lack of amplification signal would indicate that the target is not present in the sample. However, this could also be the result of failure of amplification (due to inhibition) or failure of detection due to probe failure. Probe failure could be the result of multiple mutations in the region where the probe should bind to the target or by other reasons such as loss of the fluorescent label. This condition can be recognized by the lack of signal at about 65°C or below (originating from the probe) but the presence of a melting peak at about 90°C (originating from the low amount of double stranded amplicon that is still formed during the procedure and which is detected by the intercalating dye).

In one embodiment, the region to be amplified is the promoter region of the *cyp*51A gene of *Aspergillus fumigatus.* The corresponding mutation is a 34 bp duplication.

The above described mutations are known until now but other mutations may be found in the future. The same approach can be used to detect other mutations. The skilled person can develop primers and probes for the DNA regions comprising these other mutations without undue burden. The method of the invention is thus not limited to the mutations mentioned in this application but is more broadly applicable.

The concentration of fungal DNA in blood of patients with invasive aspergillosis is likely to be very low. Since the method of the invention targets a single copy gene, this means that sometimes there may be insufficient analytical sensitivity. In a preferred embodiment, therefore, the method comprises a preparation step for determining the amount of DNA present in the sample. This can be done by performing another real-time PCR that targets a multicopy gene. Such RT-PCR is then preferably performed in a (semi)quantitative fashion to determine whether or not sufficient DNA is present to perform the assay of the invention. Such multicopy targets do not provide information about the resistance status of the isolate causing invasive aspergillosis but are used in the pretreatment. Ribosomal gene targets or mitochondrial DNA targets, both of which are present in multiple copies per cell, can be used for this. Suitable methods to establish the DNA content of the sample are known to the person skilled in the art and have for example been described by Millon et al. J Clin Microbiol 43(10):5097-5101, 2005; Challier et al. J Clin Microbiol 42(2):844-846, 2004; Costa et al. J Clin Microbiol 40(6):2224-2227, 2002; and Kami et al. Clin Infect Dis 33(9):1504-1512, 2001.

If a standard DNA isolation procedure does not yield sufficient DNA for performing the assay, larger volumes of blood could be processed. Alternatively, DNA fragments encoding part of the cyp51A gene can be selectively extracted and enriched from blood with so-called capture probes.

The invention further relates to various primer sets and probes for use in the method.

In a first embodiment the primer set is for amplification of a DNA region that potentially comprises a 34 bp promoter duplication, which primer set comprises as the forward primer:
5'-AATAATCGCAGCACCACTCC-3' **(SEQ ID NO:1)**
   and as the reverse primer
5'-TGGTATGCTGGAACTACACCTT-3' **(SEQ ID NO:2)**

The detection of these mutations can be correlated to the resistance status of the *Aspergillus fumigatus* strain that has invaded the patient and of which the DNA can be detected in blood and blood derivatives or organ samples, and in particular in serum. Thus, clinical samples are used to determine the presence of *A. fumigatus,* that can be triazole-resistant or triazole-sensitive, in that sample in a (semi-)quantitative or qualitative manner without the need for cultivation of the isolate. Thus, the diagnosis can be performed much quicker than with the currently used detection methods.

In a further embodiment, the location of either or both PCR amplification primers is shifted closer to, or further away from the target to amplify either smaller or larger products, respectively. In another embodiment, either one or both amplification primers are shorter or longer than indicated making it possible that the annealing (and or extension) reaction is performed at a different temperature. In a still further embodiment, either or both primer(s) contain modified nucleotides or nucleotide analogues or other non-natural compounds such as PNA, LNA or other chemistries.

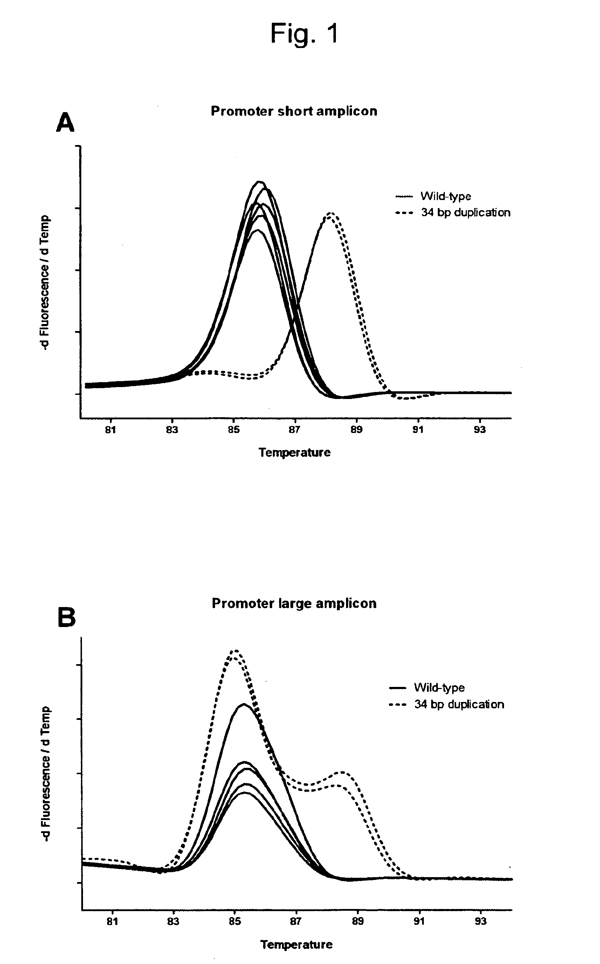

Detection of the 34-bp duplication in the *cyp*51A promoter region is for example established using the indicated PCR amplification primers. In a further embodiment, identification of the duplication is established using a combination of other amplification primers that amplify either a shorter or longer fragment by using a combination of primers that have shifted closer to the duplicated region or further away from it. As shown in **figure 1B****,** it is still possible to discriminate between the non-duplicated versus the duplicated target using melting curve analysis in a fragment of ∼500 bp. In another preferred embodiment, the same technique is used to detect duplications of other regions in the cyp51A promoter.

If a mutation has been observed, the information provided in the table below can be used to determine whether the isolate is resistant or not to one or more triazole based antifungal compounds.

The invention will be further illustrated in the Examples that follow. In the first example the various assay formats are illustrated in a model system. Example 2 relates to a real-life case demonstrating the necessity of the invention. In the examples specific primers and probes are used and specific concentrations given. It is clear for the skilled person that these and other data in the Examples are only given for illustration purposes and are in no way limiting to the Example. The skilled person in the field of amplification techniques is very well capable of making suitable modifications without departing from the scope of the invention.

In the Examples reference is made to the figures.
**Fig. 1A** shows the melting curves of a short amplicon of the promoter of the *cyp*51A gene. The continuous line represents wild type and the dotted line represents the 34-bp duplication.
**Fig. 1B** shows the melting curves of a long amplicon (about 500 bp) of the promoter of the *cyp*51A gene. The continuous line represents wild type and the dotted line represents the 34-bp duplication.
**Fig. 2A** shows the melting curves of a wild type target and a target comprising the Leu98>His mutation probed with a Leu98>His probe. The continuous line represents wild type and the dotted line represents the Leu98>His mutation.
**Fig. 2B** shows the melting curves of a wild type target and a target comprising the Leu98>His mutation probed with a wild type probe. The continuous line represents wild type and the dotted line represents the Leu98>His mutation.
**Fig. 2C** shows the melting curves of a wild type target and a target comprising the either a Gly54>Glu mutation or a Gly54>Val mutation probed with a wild type probe. The continuous line represents wild type, the dotted line represents the Gly54>Glu mutation, and the dashed line the Gly54>Val mutation.
**Fig. 2D** shows the melting curves of a wild type target and a target comprising a Met220>Val or a Met220>Lys mutation probed with a wild type probe. The continuous line represents wild type, the dotted line represents the Met220>Val mutation and the dashed line is the Met220>Lys mutation.
**Fig. 2E** shows the melting curves of wild type targets probed with a mutant probe for the Gly138>Cys mutation. The continuous line represents wild type.
**Fig. 3A** shows difference plots of a samples with the Lys98>His mutation or a different mutation using the HRM variant. The solid line represents Af293, the dashed line represents the Leu98>His mutation and the dotted line represents a silent variation at Gly89.
**Fig. 3B** shows difference plots of a samples with a mutation at Gly54 using the HRM variant. The solid line represents the reference sample, the dashed line represents a mutation at Gly54 and the dotted line represents no mutation at Gly54.
**Fig. 4** shows the melting curves of a short amplicon of the promoter of the *cyp*51A gene obtained using serum samples. The continuous lines represent wild type samples and the dotted line represents a control sample with the 34-bp duplication. The dashed line represents a patient serum sample with the 34 bp duplication.

### EXAMPLES

### EXAMPLE 1

### Introduction

This example illustrates the application of two new mutation detection formats based on a HRM dye for analysis of mutations in the *cyp*51A gene leading to triazole resistance in *Aspergillus fumigatus.* Several mutations that are causative of a resistance phenotype have been identified in this gene. Especially four positions in the CYP51A protein have unambiguously been implicated in resistance to triazole based antifungals. This involves Gly54, Met220, Gly138 and Leu98. Known so far, the latter only leads to resistance in combination with a 34 bp duplication in the promoter region of the *cyp*51A gene.

### Materials and Methods

### Reference samples

*A. fumigatus* isolates containing a variety of mutations at codons 54 or 220 of the cyp51A gene were a gift of dr. E. Mellado (Instituto de Salud Carlos III, Majadahonda, Madrid, Spain). DNA samples containing the L98H mutation and the 34bp duplication in the promoter region of the cyp51A gene were from the clinical isolate collection of the Canisius Wilhelmina Hospital, Nijmegen, the Netherlands.

Random A. *fumigatus* samples from phenotypically sensitive isolates were taken from the clinical isolates collection of the Canisius Wilhelmina Hospital as wild-type references. In addition, Af293, the strain whose genome has been sequenced, was used as a reference strain and was obtained through the Centraalbureau voor Schimmelcultures (Utrecht, The Netherlands).

### DNA isolation

DNA was isolated from sporulating cultures essentially as described elsewhere (de Valk et al., J Clin Microbiol 43(8):4112-4120, 2005

### Probe variant: targeted scanning using a single fluorescent labeled oligonucleotide probe

Amplifications were performed on a LightCycler 480 (Roche diagnostics, Almere, The Netherlands) in separate reactions for each target in a final volume of 10 *µ*l containing 1 ng of DNA, amplification primers (Eurogentec, Seraing, Belgium) and mutation detection probe (TIB Molbiol, Berlin, Germany) (concentration according table 1), 1 U FastStart Taq DNA polymerase (Roche diagnostics), 0.2 mM dNTP's, and 1x HRM Dye (Roche diagnostics) in 1x reaction buffer containing 2 mM MgCl₂ (Roche diagnostics) using the following cycling parameters: after an initial denaturation step for 10 min at 95°C, 45 amplification cycles of 2 s denaturation at 95°C, 5s annealing at 60°C and 20s extension at 72°C were applied.

**Table 1. Overview of suitable primer- and probe concentrations.**

| **CYP51A target** | **use** | **Conc. (*µ*M)** |
|---|---|---|
| Gly₅₄ | forward amplification primer | 0.3 |
| Gly₅₄ | reverse amplification primer | 0.2 |
| Gly₅₄ | probe | 0.2 |
| Leu₉₈ | forward amplification primer | 0.1 |
| Leu₉₈ | reverse amplification primer | 0.5 |
| Leu₉₈ | wild-type probe | 0.2 |
| Leu₉₈ | mutant probe | 0.2 |
| Gly₁₃₈ | forward amplification primer | 0.2 |
| Gly₁₃₈ | reverse amplification primer | 0.5 |
| Gly₁₃₈ | probe | 0.2 |
| Met₂₂₀ | forward amplification primer | 0.5 |
| Met₂₂₀ | reverse amplification primer | 0.2 |
| Met₂₂₀ | probe | 0.2 |

The melting protocol consisted of denaturation at 95°C for 2s and 45°C for 30s after which the temperature was increased to 95°C with 10 acquisitions per °C under continuous monitoring of the fluorescence.

### HRM variant: Real-Time scanning for mutations without fluorescent oligonucleotides

Amplification reactions were performed as above with the following modifications. First, the mutation detection probe was omitted from the reaction mixtures and second, 1 ng of reference DNA was added to each amplification reaction. Primers were present in equimolar amounts (0.5 *µ*M each). Heteroduplexes were created post-amplification by denaturation for 5 s at 95°C, annealing for 30 s at 60°C followed by melting analysis using 25 acquisitions per °C up to 95°C. Melting data was analyzed using the LC480 Gene Scanning software as recommended by the manufacturer(Roche diagnostics).

### Detection of the 34-bp duplication in the cyp51A promotor region

Amplifications were performed in a reaction volume of 10 *µ*l using 1 ng of genomic DNA, 0.5 *µ*M of amplification primers 51APrF and 51APrR, 0.5 U FastStart Taq DNA polymerease (Roche), 0.2 mM dNTP's, 1x HRM dye and 2.5 mM MgCl₂ in 1x reaction buffer.

The amplification parameters were denaturation 10 min at 95°C followed by 35 cyles of 95°C for 1 sec, 60°C for 2s and 72°C for 5s.

Following amplification, melting curves were recorded by increasing the temperature up to 95°C with 5 acquisitions per °C.

### DNA sequence analysis

Obtained PCR products were purified using SPRI chemistry (Agencourt, Beverly, MA, USA) and subjected to bidirectional sequence analysis using the respective amplification primers with the use of the DYEnamic ET Dye Terminator Kit according to the recommendations of the manufacturer (GE Healthcare, Diegem, Belgium).

Reaction products were purified using SPRI chemistry and analyzed on a MegaBACE 500 automated DNA analysis platform equipped with a 48 capillary array as recommended (GE Healthcare).

### Results

### Probe variant: targeted scanning for known point-mutations with a single fluorescent probe

In figure 2A, B, C, D and E, melting peaks are shown for analysis of each of the four codons implicated in the triazole resistance phenotypes. In figure 2B, C and D, all probes are directed at the wild-type sequence. If no sequence variation is present, a melting peak is expected usually in the range of 65-70°C (dependent on the exact reaction conditions and probe sequence). A mutation in the region covered by the probe becomes apparent by melting peaks that have shifted to a lower temperature (usually in the range of 50-65°C) as shown for the G54E, G54V, L98H, M220V and M220K mutations. When a probe is used that is directed at the mutant sequence, the wild-type product will have the melting peak at the lower temperature and the mutated product will give rise to a melting peak at the higher temperature as shown in figure **2A** and **2E****.**

An additional melting peak is apparent for all of the above examples in the region of 80-90°C. This is the result of the formation of a relatively low amount of double-stranded PCR product (limited by the non-template primer) that is detected through the presence of the HRM dye. Amplification in symmetrical fashion yields substantially lower signals in the probe region (55-70°C) but will increase the fluorescent signals at 80-90°C.

As shown in **figure 2C****,** at the G54 position, the presence of a mutation can also be deduced from the shape of the melting curve resulting from the double stranded amplicon, thus illustrating that use of a probe is not always necessary to determine the presence of a mutation from.

### HRM variant: detecting mutations using High Resolution Melting

In **figure 3a****,** difference plots are shown for samples without the L98H mutation and matching the published cyp51A sequence (Mellado et al., J Clin Microbiol 39(7):2431-2438, 2001) (dotted line) and for samples with the L98H mutation (dashed line). To create heteroduplexes, all samples were spiked with Af293 DNA. As a reference sample, Af293 DNA was included (solid line). Samples containing a mutation can be identified based on their aberrant melting profile compared to the reference sample (visualized in the difference plots). When the samples containing the L98H mutation were compared to the reference DNA sample, difference plots were obtained with a markedly different shape compared to the reference sample. Also samples with a different variation (a silent mutation at Gly89) showed significant differences relative to the Af293 sample as well as to the samples with a Leu98>His mutation.

In **figure 3B****,** a similar example is shown as above, but now for the G54 position. The dotted lines represent samples with a wild-type cyp51A sequence and these do not significantly deviate from the reference sample. The dashed lines represent samples with a mutation at the G54 position. All samples were spiked with a reference sample matching the published cyp51A sequence.

### Amplicon variant: Real-time detection of the cyp51A promoter polymorphism

In **figure 1A** and B typical amplification curves and melting peaks are shown for the *cyp*51A promoter region using either short or long amplification products, respectively. In **figure 1A****,** presence of the 34 bp duplication in the *cyp*51A promoter will lead to the generation of a significantly larger PCR product (139 versus 105 bp, respectively) that is easily distinguished from the wild-type product by its melting peak at increased temperatures (ca. 88 versus ca. 86°C. respectively).

In control amplification reactions without template DNA, no melting peaks were obtained, indicating the lack of formation of non-specific PCR products (not shown).

### EXAMPLE 2

### Use of the assay of the invention to test serum of a patient

A female patient was diagnosed with invasive aspergillosis. She received standard antifungal treatment. Sputum samples were collected on a frequent basis to look for fungal isolates. Blood samples were also frequently withdrawn from the patient. Despite treatment the patient died.

Retrospectively, all cultivated *Aspergillus fumigatus* isolates were phenotypically tested for resistance. In the first weeks, only triazole sensitive isolates were cultured but as of week 5 multi-triazole resistant isolates were also found. Stored blood samples from the patient were also analyzed for the presence of the Tandem Repeat (TR) duplication in the *cyp*51A promoter region using the method described in this invention (table).

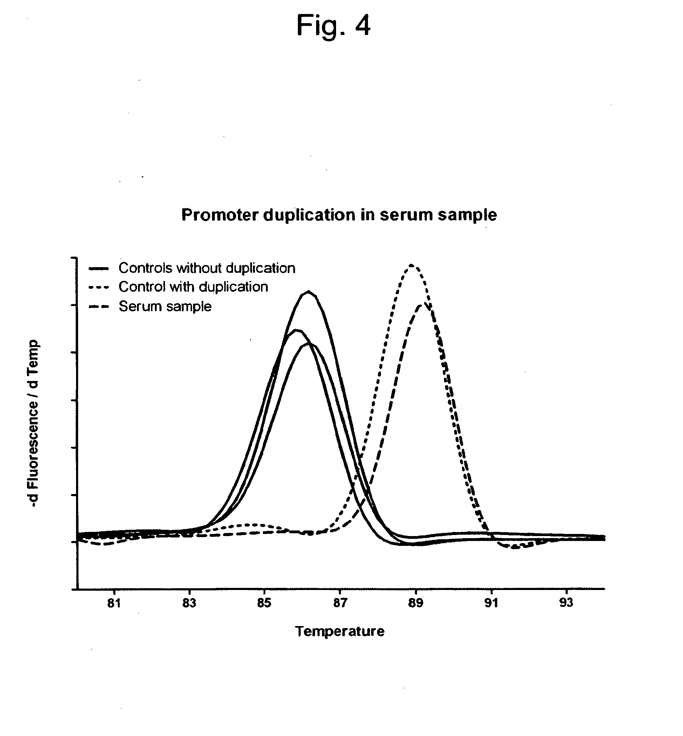

A serum sample that was collected 2 weeks before a multi-triazole resistant isolate appeared in sputum samples, tested positive for the TR duplication **(****figure 4****).** The phenotypically resistant isolates also contained the TR duplication as well as the L98H mutation. Molecular fingerprinting of this resistant isolate showed that this was a different isolate than the sensitive isolate that was cultured before. If the serum samples had been routinely analyzed for the presence of mutations leading to multi-triazole resistance, it could have been established 2 weeks earlier than by conventional culture that the patient was infected with a multi-triazole resistant isolate. If this had been done at that time, the patient could have been treated otherwise and she may have survived the infection.

## Claims

1. Method for detecting mutations in *Aspergillus fumigatus* DNA in a body sample consisting of blood or a blood derivative, comprising the steps of:
a) providing a body sample;
b) amplifying a target nucleic acid that is present in the sample, using a primer set for amplification of a DNA region of *Aspergillus fumigatus* that potentially comprises a 34 bp promoter duplication, which primer set comprises the forward primer:
5'-AATAATCGCAGCACCACTCC-3' **(SEQ ID NO:1)**
and the reverse primer
5'-TGGTATGCTGGAACTACACCTT-3' **(SEQ ID NO:2);**
c) allowing double stranded nucleic acid to form between the amplified target nucleic acid and a nucleic acid that is at least partially complementary to the target nucleic acid in the presence of a fluorescent compound, the fluorescence of which is enhanced or altered upon intercalation of double stranded nucleic acid;
d) melting the double stranded nucleic acid thus formed and measuring the fluorescence in the sample as a function of the temperature to obtain a melting curve, and
e) analysing the melting curve to detect mutations in the target nucleic acid sequence; and **characterized in that** the nucleic acid that is at least partially complementary to the target nucleic acid is an amplified reference nucleic acid corresponding to the wild type fungal or yeast DNA.

2. Method as claimed in claim 1, wherein the body sample serum.

3. Method as claimed in any one of the claims 1-2, wherein the fluorescent compound is a dye selected from the HRM dye, Sybr-Green, LC-Green, LC-Green plus, SYTO-dyes.

4. Method as claimed in any one of the claims 1-3, wherein the nucleic acid that is at least partially complementary to the target nucleic acid is a fluorescent label, that is excited by the fluorescence emitted by the fluorescent compound upon intercalation thereof in double stranded nucleic acid.

5. Method as claimed in claim 4, wherein the oligonucleotide probe is complementary to a region on the target nucleic acid suspected to contain a mutation.

6. Method as claimed in claim 5, wherein the region is the promoter region of the *cyp*51A gene of *Aspergillus fumigatus.*

7. Kit for performing the method as claimed in any one of the claims 1-6, which kit comprises at least one primer set as defined in claim 1, for the amplification of a target region in the CYP51A gene of *Aspergillus fumigatus,* a nucleic acid sequence that is at least complementary to the target region and optionally a protocol explaining the method to be followed.

## Patentansprüche

1. Ein Verfahren zur Erkennung von Mutationen der DNA von *Aspergillus fumigatus* in - aus Blut oder Blutderivaten bestehenden - Körperproben, welches die folgenden Schritte umfasst:
a) Herstellung einer Körperprobe;
b) Amplifizierung einer in der Probe vorhandenen Zielnukleinsäure unter Verwendung eines Primersets zur Amplifizierung einer DNA-Region von *Aspergillus fumigatus* ggf. mit einer 34-bp-Promoterverdoppelung, wobei das betreffende Primerset den Vorwärtsprimer
5'-AATAATCGCAGCACCACTCC-3' **(SEQ ID Nr.1)**
sowie den Rückwärtsprimer
5'-TGGTATGCTGGAACTACACCTT-3' **(SEQ ID Nr.2)**
enthält;
c) die Zulassung der Bildung einer doppelsträngigen Nukleinsäure zwischen der amplifizierten Zielnukleinsäure und einer Nukleinsäure, welche sich in der Gegenwart einer fluoreszierenden Verbindung zumindest teilweise komplementär verhält, wobei die Fluoreszenz der betreffenden Verbindung im Zuge der Interkalation der doppelsträngigen Nukleinsäure gesteigert oder modifiziert wird;
d) die Schmelzung der entsprechend gebildeten doppelsträngigen Nukleinsäure und die Messung der Fluoreszenz in der Probe als Funktion der Temperatur mit dem Ziel, eine Schmelzkurve zu erstellen, und
e) die Analyse der Schmelzkurve mit dem Ziel, Mutationen in der Sequenz der Zielnukleinsäure zu erkennen; und **dadurch gekennzeichnet, dass** es sich bei der Nukleinsäure, welche sich zumindest teilweise komplementär zu der Zielnukleinsäure verhält, um eine amplifizierte Referenznukleinsäure handelt, die der Wildtyp-DNA von Pilzen oder Hefe entspricht.

2. Das Verfahren nach Anspruch 1, wobei es sich bei der Körperprobe um Serum handelt.

3. Das Verfahren nach einem der Ansprüche 1 oder 2, wobei es sich bei der fluoreszierenden Verbindung um einen Farbstoff aus der folgenden Gruppe handelt: HRM-Farbstoffe, Sybr-Green, LC-Green, LC-Green plus, SYTO-Farbstoffe.

4. Das Verfahren nach einem der Ansprüche 1 bis 3, wobei es sich bei der Nukleinsäure, welche sich zumindest teilweise komplementär zu der Zielnukleinsäure verhält, um einen nicht-fluoreszierenden Marker handelt, der durch die Fluoreszenz angeregt wird, welche von der fluoreszierenden Verbindung bei deren Interkalation in die doppelsträngige Nukleinsäure emittiert wird.

5. Das Verfahren nach Anspruch 4, wobei sich die Oligonukleotidprobe komplementär zu einer Region der Zielnukleinsäure verhält, in welcher eine Mutation vermutet wird.

6. Das Verfahren nach Anspruch 5, wobei es sich bei der Region um die Promoterregion des Gens *cyp*51A von *Aspergillus fumigatus* handelt.

7. Ein Kit zur Durchführung des Verfahrens nach einem der Ansprüche 1 bis 6, welches mindestens einen Primer gemäß der entsprechenden Definition von Anspruch 1 zur Amplifizierung einer Zielregion des Gens CYP51A von *Aspergillus fumigatus* enthält, ebenso eine Nukleinsäuresequenz, die sich zumindest teilweise komplementär zu der Zielregion verhält, und wahlweise ein Protokoll, welches das auszuführende Verfahren beschreibt.

## Revendications

1. Une méthode pour détecter les mutations dans l'ADN de *l'Aspergillus fumigatus* dans un échantillon corporel de sang ou de dérivés de sang, comprenant les étapes suivantes :
a) fournir un échantillon corporel ;
b) amplifier un acide nucléique cible qui se trouve dans l'échantillon, en utilisant un ensemble d'amorces pour l'amplification de la région ADN de l' *Aspergillus fumigatus* qui pourrait comprendre une duplication du promoteur de 34 bp dans l'ensemble d'amorces qui comprend l'amorce avant :
5'-AATAATCGCAGCACCACTCC-3' **(SEQ ID NO : 1)** et l'amorce inverse
5'-TGGTATGCTGGAACTACACCTT-3' **(SEQ ID NO : 2) ;**
c) permettre à un acide nucléique double brin de former entre l'acide nucléique cible amplifié et l'acide nucléique qui est au moins partiellement complémentaire à l'acide nucléique cible en présence d'un composé fluorescent, la fluorescence est améliorée ou modifiée selon l'intercalation de l'acide nucléique à double brin ;
d) fusion de l'acide nucléique double brin ainsi formé et mesure de la fluorescence dans l'échantillon comme une fonction de la température afin d'obtenir une courbe de fusion, et
e) analyser la courbe de fusion afin de détecter les mutations dans la séquence de l'acide nucléique cible ; et **caractérisé en ce que** l'acide nucléique soit au moins partiellement complémentaire à l'acide nucléique cible et qu'il soit une référence amplifiée de l'acide nucléique correspondant au type sauvage de l'ADN fongique et de la levure.

2. Une méthode selon la revendication 1, avec le sérum de l'échantillon corporel.

3. Une méthode selon les revendications 1-2, où le composé fluorescent est un colorant sélectionné dans les colorants HRM, Sybr-Green, LC-Green, LC-Green plus, colorants SYTO.

4. Une méthode selon les revendications 1-3, où l'acide nucléique qui est au moins partiellement complémentaire à l'acide nucléique cible est un marqueur fluorescent, qui est excité par la fluorescence émise par le composé fluorescent sur l'intercalation de l'acide nucléique double brin.

5. Une méthode selon la revendication 4, ou la sonde oligonucléotique est complémentaire à la région de l'acide nucléique cible susceptible de contenir une mutation.

6. Une méthode selon la revendication 5, ou la région est la région promotrice du gène *cyp*51A de l'*Aspergillus fumigatus.*

7. Un ensemble pour exécuter la méthode selon les revendications 1-6, dont l'ensemble comprend au moins un ensemble d'amorces comme il est défini dans la revendication 1, pour l'amplification de la région cible dans le gène CYP51A de l'Aspergillus *fumigatus,* une séquence d'acide nucléique qui est au moins complémentaire à la région cible et éventuellement un protocole expliquant la méthode à suivre.
